# EUROPEAN PATENT SPECIFICATION

(11) **EP 0 645 373 B1**
(45) Date of publication and mention of the grant of the patent: **05.11.1997**
(21) Application number: 94112187.3
(22) Date of filing: 01.08.1994
(51) Int. Cl.: C07C 291/08, C07D 319/08, C07D 317/66

(54) **Process for the preparation of azoxycyanide compounds**
Verfahren zur Herstellung von Azoxycyanid-Verbindungen
Procédé de préparation de composés d'azoxycyanide

(30) Priority: 18.08.1993 EP 93113191
(43) Date of publication of application: 29.03.1995
(73) Proprietor: SHELL INTERNATIONALE RESEARCH MAATSCHAPPIJ B.V., 2596 HR Den Haag (NL)
(72) Inventor: Simon, Werner Emil Josef, D-74076 Heilbronn (DE)
(74) Representative: Wächtershäuser, Günter, Prof. Dr.

(56) References cited:
- CHEMICAL ABSTRACTS, vol. 114, no. 21, 27 May 1991, Columbus, Ohio, US; abstract no. 207072t, S. G. ZLOTIN ET AL. page 813 ; & IZV. AKAD. NAUK. SSSR KHIM., no.12, 1990 pages 2821 - 2826
- JOURNAL OF THE CHEMICAL SOCIETY, CHEMICAL COMMUNICATIONS, 1984, LETCHWORTH GB pages 323 - 324 R. FRUTTERO ER AL. 'A direct synthesis of alkyl, aryl, and heteroaryl-ONN-azoxycyanides'

## Description

This invention relates to a process for the preparation of certain azoxycyanide compounds, especially arylazoxycyanide compounds which exhibit biocidal activity.

A number of routes for the preparation of arylazoxycyanide compounds have already been disclosed. For instance, Eur. J. Med. Chem. - Chim. Ther., 1982, 17, pp. 482-484 discloses the oxidation of an arylazocyanide under harsh conditions using 90% hydrogen peroxide in trifluoroacetic acid. However, such a route would be hazardous and expensive for the large scale preparation of arylazoxycyanide compounds.

European patent application no. 92120580.3 discloses a modification of the above oxidation process in which an arylazocyanide is oxidised under milder conditions with a mixture comprising hydrogen peroxide and methanoic acid and/or with peroxymethanoic acid. However, as this modified process still uses potentially hazardous materials, it is not suitable for use on a commercial scale.

Another route, described in J. Chem. Soc., Chem. Commun., 1984, pp 323-324, involves the reduction of an aromatic nitro group to a hydroxylamine followed by oxidation back to a nitroso compound and, in turn, coupling with cyanamide using iodobenzene diacetate in stoichiometric amounts. A severe disadvantage of this route for large scale preparation of arylazoxycyanide compounds is the requirement for stoichiometric amounts of iodobenzene diacetate. Iodobenzene diacetate is not available in large quantities and this adds significantly to the costs of the preparation.

A modification of the above process is described in European patent application no. 92120580.3 which uses dibromoisocyanuric acid in place of iodobenzene diacetate. However, although dibromoisocyanuric acid is cheaper to produce than iodobenzene diacetate, it is not commercially available. Moreover, in large scale preparation, dibromoisocyanuric acid causes the release of elemental bromine which partially oxidises the nitroso precursor to nitroaromatic compounds and the final product is therefore always contaminated with differing amounts of nitro compounds.

It has now been discovered that arylazoxycyanide compounds can be produced in high yield from the corresponding nitroso compound using reagents that are commercially available at reasonable prices. Moreover, since these reagents do not appear to cause the release of elemental bromine, the formation of undesired by-products is greatly reduced.

According to the present invention there is therefore provided a process for the preparaton of a compound of the general formula 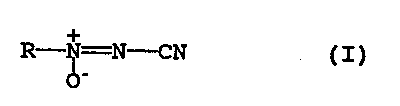 in which R represents an optionally substituted aryl or heterocyclyl group, which comprises treating a compound of the general formula

R-N=O (II)

in which R is as defined above, with cyanamide or a metal salt thereof and a compound of the general formula 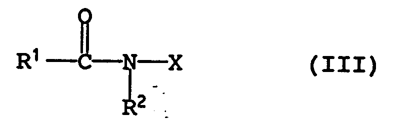 in which R¹ represents an optionally substituted alkyl or aryl group and R² represents a hydrogen atom or an optionally substituted alkyl or aryl group or R¹ and R² together represent a group 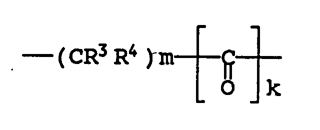 where m is 2, 3, 4 or 5, k is 0 or 1 and each of R³ and R⁴ independently represents a hydrogen atom or an alkyl group, and X represents a chlorine, bromine or iodine atom or a cyano or -SO₂R⁵ group where R⁵ represents a hydrogen atom or an optionally substituted alkyl or aryl group.

It should be noted that compounds of general formula I could be in any of the following isoelectronic forms: 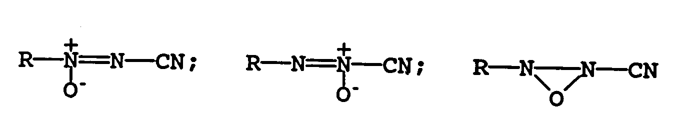 and the scope of the present invention covers all such forms.

Unless otherwise stated, when any of the compounds mentioned contain an alkyl, alkenyl or alkynyl substituent group, this may be linear or branched and may contain up to 12, preferably up to 6 and especially up to 4, carbon atoms. Cycloalkyl or cycloalkenyl groups may contain 3 to 8, preferably 3 to 6, carbon atoms. An aryl group may be any aromatic hydrocarbon group, especially a phenyl or naphthyl group. An aralkyl group comprises an alkyl group, as defined above, substituted by an aryl group, as defined above. A particularly preferred example of an aralkyl group is a benzyl group. A heterocyclyl group may be any saturated or unsaturated ring system containing at least one heteroatom, 3- to 6-membered rings being preferred and 5- and 6-membered rings being especially preferred. Nitrogen-, oxygen- and sulphur-containing heterocyclic rings, such as pyridyl, pyrimidinyl, pyrrolidinyl, furyl, pyranyl, morpholinyl and thienyl, are particularly preferred.

When any of the foregoing substituents are designated as being optionally substituted, the substituent groups which are optionally present may be any one or more of those customarily employed in the development of pesticidal compounds, and/or the modification of such compounds to influence their structure/activity, persistence, penetration or other property. Specific examples of such substituents include, for example, halogen atoms, nitro, cyano, hydroxyl, alkyl, haloalkyl, alkoxy, haloalkoxy, amino, alkylamno, dialkylamino, formyl, alkoxycarbonyl, carboxyl, alkanoyl, alkylthio, alkylsulphinyl, alkylsulphonyl, carbamoyl and alkylamido groups. When any of the foregoing substituents represents or contains an alkyl substituent group, this may be linear or branched and may contain up to 12, preferably up to 6, and especially up to 4, carbon atoms. Typically, 0-3 substituents may be present, most commonly 0 or 1.

The process described herein may be particularly, although not exclusively, of use in the preparation of the following groups of compounds.

In one group of compounds of interest, R represents a substituted phenyl group in which at least one substituent thereof is a group of general formula -NR⁶COR⁷ where R⁶ represents a hydrogen atom or an alkyl group and R⁷ represents a hydrogen atom or an optionally substituted alkyl, alkoxy, alkenyl, alkenyloxy, phenyl or phenoxy group. Preferably, R⁶ represents a hydrogen atom. Preferably, R⁷ represents a hydrogen atom or an optionally substituted alkyl, alkenyl, phenyl or alkoxy group. Preferably R⁷ represents a hydrogen atom, or an alkyl, haloalkyl (especially CF₃), alkoxy or phenyl group. Most preferably, a group -NR⁶COR⁷ is of formula -NHCOC₆H₅. Preferably, the phenyl group R carries only one substituent, preferably in the 2- or 4- position.

In another group of compounds of interest, R represents a substituted phenyl group in which at least one substituent thereof is an amido group, preferably of general formula -CONR⁸R⁹ where each of R⁸ and R⁹ independently represents a hydrogen atom or an optionally substituted (preferably unsubstituted) alkyl group, or an optionally substituted phenyl group, preferably phenyl optionally substituted by one or more halogen atoms(s) and/or alkyl group(s). Preferably, each of R⁸ and R⁹ independently represents a hydrogen atom, a C₁₋₄ alkyl group, or an optionally halo-substituted phenyl group. Preferably, at least one of R⁸ and R⁹ represents a hydrogen atom, and the other of R⁸ and R⁹ represents a hydrogen atom, methyl group, phenyl group or halophenyl group. Preferably, the phenyl group R carries only one substituent, preferably in the 2- or, more preferably, the 4-position.

In another group of compounds of interest, R may represent a group of general formula. 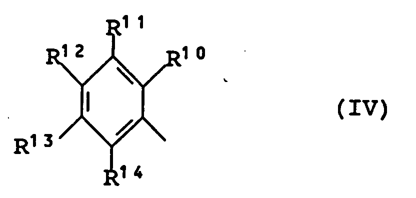 where R¹⁰ and R¹¹ together, or R¹¹ and R¹² together, represent an optionally substituted hydrocarbyloxy chain and the ring is optionally substituted at any or each of the remaining sites R¹³, R¹⁴ and R¹⁰ or R¹².

The term "hydrocarbyloxy chain" is used herein to denote a carbon chain interrupted within the chain by one or more (but preferably one only) oxygen atom. A (or the) oxygen atom is preferably located at one end of the chain.

Optional substituent(s) of the hydrocarbyloxy chain are, suitably, optionally substituted alkyl group(s), preferably alkyl group(s) optionally substituted by one or more (preferably one) halogen atom(s) or hydroxy or alkoxy group(s); optionally substituted phenyl group(s); an alkylene group, preferably -(CH₂)₄-, across adjacent carbon atoms of the hydrocarbyloxy chain; or group(s) =O.

Preferably, the hydrocarbyloxy chain is unsubstituted or substituted by 1-2 alkyl, haloalkyl, hydroxyalkyl or alkoxyalkyl groups, or by one alkylene group -(CH₂)₄- alkoxyalkyl groups, or by one alkylene group -(CH₂)₄- across adjacent carbon atoms, or by a group =O.

A hydrocarbyloxy chain preferably has 3 or 4 chain atoms. Preferred hydrocarbyloxy chains may be represented by oxyalkylene and oxyalkenylene chains. Within the ambit of the term "oxyalkenylene" as used herein are chains in which the pi electrons form part of a resonance electron system. When the chain is oxyalkenylene the chain may form, with the phenyl ring, a fused heteroaromatic ring system.

Preferred oxyalkylene and oxyalkenylene chains are based upon these structures (side (atoms/groups not shown):
-O-C-C- (cf dihydrobenzo[b]furans)
-O-C=C (cf benzo[b]furans)
-O-C-C-C-
-O-C-C=C-

Particularly preferred oxyalkylene or oxyalkenylene chains are: 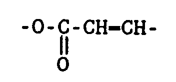 -O-C(CH₃)=CH-
-O-C(CH₃)₂-CH₂-
-O-CH₂-CH=CH-

Optional substituents R¹³, R¹⁴ and R¹⁰ or R¹² are preferably selected from halogen atoms and alkyl and alkoxycarbonyl groups, especially chlorine, methyl and methoxycarbonyl. Most preferably, the sites R¹³, R¹⁴ and R¹⁰ or R¹² are all unsubstituted, or only one such site is substituted.

In another group of compounds of interest, R may represent a group of general formula 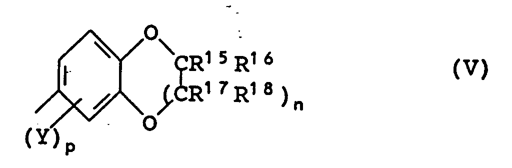 wherein n is 0 or 1; each of R¹⁵ and R¹⁶, and R¹⁷ and R¹⁸ if present, independently represents a hydrogen or halogen atom or an optionally substituted alkyl, cycloalkyl or aryl group, or R¹⁵ and R¹⁶ together or R¹⁷ and R¹⁸ together represent an optionally substituted alkylene chain; Y represents an alkyl group or a halogen atom; and p represents 0, 1, 2 or 3. Preferably each of R¹⁵ and R¹⁶ independently represents a hydrogen atom or a C₁₋₆ alkyl, C₃₋₆ cycloalkyl, phenyl or naphthyl group or R¹⁵ and R¹⁶ together represent a C₄₋₆ alkylene chain, each group or chain being optionally substituted by one or more substituents selected from halogen atoms, nitro, cyano, hydroxyl, C₁₋₄ alkyl, C₁₋₄ haloalkyl, C₁₋₄ alkoxy, C₁₋₄ haloalkoxy, C₁₋₄ alkylamino, di-C₁₋₄ alkylamino, C₁₋₄ alkoxycarbonyl and benzyloxycarbonyl groups. Preferably, each of R¹⁷ and R¹⁸, if present, represents a hydrogen atom or a C₁₋₆ alkyl, C₃₋₆ cycloalkyl, phenyl or naphthyl group or R¹⁷ and R¹⁸ together represent a C₄₋₆ alkylene chain, each group or chain being optionally substituted by one or more substituents selected from halogen atoms, nitro, cyano, hydroxyl, C₁₋₄ alkyl, C₁₋₄ haloalkyl, C₁₋₄ alkoxy, C₁₋₄ haloalkoxy, C₁₋₄ alkylamino, di-C₁₋₄ alkylamino, C₁₋₄ alkoxycarbonyl and benzyloxycarbonyl groups. Preferably, Y represents a fluorine or chlorine atom. Preferably p represents 0.

In another group of compounds of interest, R may represent a substituted phenyl group in which at least one substituent thereof is a group of general formula -S(O)_{q}Z wherein q represents 0, 1 or 2 and Z represents a hydrogen atom or an optionally substituted alkyl, cycloalkyl, aryl, aralkyl or amino group. More specficially, Z preferably represents an optionally substituted alkyl, cycloalkyl, aralkyl or aminio group. More preferably, Z represents a C₁₋₄ alkyl group, a C₃₋₆ cycloalkyl group, a benzyl group or a group of general formula NR²⁹R³⁰ where each of R²⁹ and R³⁰ independently represents a hydrogen atom, or an optionally substituted phenyl or alkyl group, or together may represent an alkylene chain which is optionally interrupted by an oxygen atom. Preferably, R²⁹ represents a hydrogen atom and R³⁰ represents a hydrogen atom, or a phenyl or halophenyl group, or an alkyl group optionally substituted by a group of general formula -CO₂R³¹ where R³¹ represents a hydrogen atom or an alkyl group; or R²⁹ and R³⁰ together represent a group of formula -(CH₂)₂-O-(CH₂)₂- (the group Z thus being an N-morpholino group). Preferably, the phenyl group R is substituted by one or more further substituents for example halogen atoms(s). Preferably, R represents a group of general formula 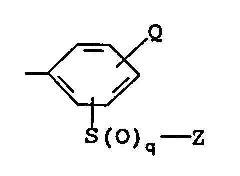 where Q represents a halogen, especially chlorine, atom, or most preferably, a hydrogen atom.

In another group of compounds of interest, R represents a group of general formula 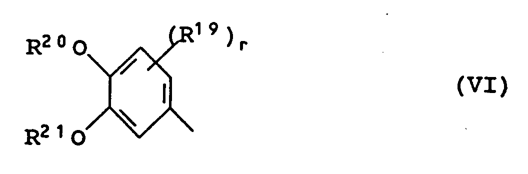 in which r is 0, 1, 2 or 3; each R¹⁹ independently represents a halogen atom, nitro, cyano, alkyl, haloalkyl, alkoxy or haloalkoxy group; and each of R²⁰ and R²¹ independently represents an optionally subsituted alkyl, alkenyl, alkynyl, cycloalkyl or aralkyl group. Preferably, each R¹⁹ independently represents a halogen (especially fluorine or chlorine) atom, nitro, cyano, C₁₋₆ alkyl (especially methyl), C₁₋₆ haloalkyl (especially trifluoromethyl), C₁₋₆ alkoxy or C₁₋₆ haloalkoxy group. It is preferred that r is 0 or 1 and especially preferred that r is 0. Preferably, each of R²⁰ and R²¹ independently represents a C₁₋₈ alkyl, C₂₋₆ alkenyl, C₂₋₆ alkynyl, C₃₋₈ cycloalkyl or benzyl group, each group being optionally substituted by one or more substituents selected from halogen atoms, nitro, cyano, hydroxyl, C₁₋₄ alkyl, C₁₋₄ haloalkyl, C₁₋₄ alkoxy, C₁₋₄ haloalkoxy, amino, C₁₋₄ alkylamino, di-C₁₋₄ alkylamino and C₁₋₄ alkoxycarbonyl groups.

In another group of compounds of interest, R represents a group of general formula 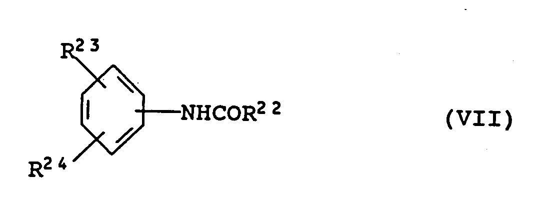 in which R²² represents an optionally substituted alkyl, alkenyl, alkynyl, cycloalkyl, cycloalkenyl, aryl or heterocyclyl group; R²³ represents a halogen atom, nitro, cyano or optionally subsituted alkyl, alkoxy, aryl or aryloxy group; and R²⁴ represents a hydrogen or halogen atom, nitro, cyano or optionally substituted alkyl or alkoxy group. Preferably, R²² represents a C₁₋₆ alkyl, C₂₋₆ alkenyl, C₂₋₆ alkynyl, C₃₋₈ cycloalkenyl or phenyl group or 3- to 6-membered heterocyclic ring (especially an oxygen-containing ring), each group or ring being optionally substituted by one or more substituents selected from halogen (especially fluorine and chlorine) atoms, nitro, cyano, hydroxyl, C₁₋₄ alkyl, C₁₋₄ haloalkyl, C₁₋₄ alkoxy, C₁₋₄ haloalkoxy, amino, C₁₋₄ alkylamino, di-C₁₋₄ alkylamino, C₁₋₄ alkoxycarbonyl and C₃₋₈ cycloalkyl groups. It is preferred that R²³ represents a halogen (especially chlorine) atom, a nitro or cyano group, or a C₁₋₆ alkyl (especially methyl) or C₁₋₆ alkoxy (especially methoxy) group each optionally substituted (but preferably unsubstituted) by one or more substituents selected from halogen (especially fluorine or chlorine) atoms, nitro, cyano, hydroxyl and amino groups. It is also preferred that R²⁴ represents a hydrogen or halogen (especially chlorine) atom. Preferably, the group -NHCOR²² is attached to the 3- or, especially, 4-position of the benzene ring in the azoxycyanobenzene moiety.

In another group of compounds of interest, R represents a group of general formula 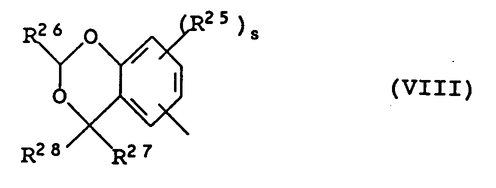 in which s is 0, 1 or 2; each R²⁵, if present, independently represents a halogen atom or an optionally substituted alkyl or alkoxy group; R²⁶ represents a hydrogen atom or a haloalkyl group; and R²⁷ and R²⁸ independently represent a hydrogen atom or an alkyl or haloalkyl group. Preferably, s is 0 or 1 and each R²⁵, if present, represents a halogen (especially chlorine or bromine) atom or a C₁₋₆ alkyl (especially methyl) or C₁₋₆ alkoxy (especially methoxy) group, each group being optionally substituted by one or more substituents selected from halogen atoms, nitro, cyano, hydroxyl and amino groups. It is preferred that R²⁶ represents a hydrogen atom or a C₁₋₆ haloalkyl group, the or each halogen atom in the haloalkyl group preferably being a fluorine or chlorine atom. It is also preferred that R²⁷ and R²⁸ independently represent a hydrogen atom or a C₁₋₆ alkyl or C₁₋₆ haloalkyl group, the or each halogen atom in the haloalkyl group preferably being a fluorine or chlorine atom. Preferably, the azoxycyano group is located at the 5-, 6- or 7-position of the 1,3-benzodioxane ring, the 6-position being especially preferred.

If a metal salt of cyanamide is used in the process of the invention, it is preferred that the metal salt is an alkali metal salt or an alkaline earth metal salt. Alternatively, such metal salts can be generated in situ by reacting cyanamide with an alkali metal hydroxide or an alkaline earth metal hydroxide. The use of monosodium cyanamide is especially preferred. Alternatively, this may be replaced by a concentrated aqueous solution of cyanamide with sodium hydroxide which effectively generates monosodium cyanamide in situ.

The compound of formula III may be a cyclic or linear N-halogen or pseudohalogen amide or imide. In the case of linear compounds, it is preferred R¹ represents a C₁₋₆ alkyl, particularly a C₁₋₄ alkyl and especially a methyl, group and R² represents a hydrogen atom. In the case of cyclic compounds, it is preferred that R¹ and R² together represent a group 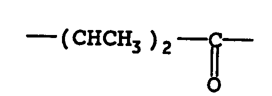 or, especially 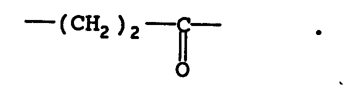 It is also preferred that X represents an iodine or, especially, a chlorine or bromine atom. If X represents a group -SO₂R⁵, it is preferred that R⁵ represents a C₁₋₄ alkyl, especially a methyl, group or a tolyl group. Particularly preferred compounds of formula III are N-chlorosuccinimide and, especially, N-bromosuccinimide.

The process is preferably carried out at a temperature in the range from -5°C to 35°C, a temperature in the range from 0°C to ambient temperature (about 20°C) being especially preferred.

The reaction may be conveniently carried out in the presence of a solvent. Suitable solvents include dimethylformamide and chlorinated hydrocarbons such as dichloromethane.

If monosodium cyanamide (or a generator thereof) and N-bromosuccinimide are used, the N-bromosuccinimide should be added to the nitroso compound of formula II before the addition of the monosodium cyanamide to avoid the production of undesired adducts between the cyanamide and the nitroso compound.

Compounds of formula II may be prepared according to the process of R.J.W. LeFevre and H. Vine, J. Chem. Soc., 1938, 431. Compounds of formula III are known compounds or may be prepared by processes analogous to known processes.

The invention also extends to compounds of general formula I whenever prepared by a process as described herein.

Certain compounds of general formula I exhibit biocidal, especially fungicidal, activity. Many of the compounds exhibit particularly good activity against phytopathogenic fungi.

The invention is further illustrated by the following examples.

### Example 1

### Preparation of [4-(N-phenylamido)phenyl]-ONN-azoxycyanide (R=4-CONHC₆H₅ phenyl)

4-(N-phenylamido)nitrosobenzene (2.7 g, 11.9 mmol) was suspended in dimethylformamide (65 ml). N-bromosuccinimide (2.17 g, 12.2 mmol) was added at once. The reaction mixture was treated in portions with monosodium cyanamide (1.18 g, 18.4 mmol). After 30 minutes at ambient temperature, the mixture was poured on water (700 ml) and the yellow solid filtered, washed with water and dried to give 2.41 g [4-(N-phenylamido)phenyl]-ONN-azoxycyanide as yellow crystals, m.pt. 220-225°C (Yield : 76.0% of theoretical).

### Example 2

### Preparation of [4-(N-phenylamido)phenyl]-ONN-azoxycyanide (R=4-CONHC₆H₅ phenyl)

4-(N-phenylamido)nitrosobenzene (5.0 g, 22.1 mmol) was suspended in dimethylformamide (50 ml) and N-bromosuccinimide (5.9 g, 33.2 mmol) was added at once. At ambient temperature, the reaction mixture was treated drop by drop with a solution of monosodium cyanamide (2.1 g, 32.8 mmol) in water (4 ml). After 20 minutes, the mixture as poured on ice-water (200 ml). The resulting yellow solid was filtered, washed with water and air dried to give 5.3 g [4-(N-phenylamido)phenyl]-ONN-azoxycyanide as a yellow powder, m.pt. 220-225°C. (Yield: 90.1% of theoretical).

### Example 3

### Preparation of [4-(N-phenylamido)phenyl]-ONN-azoxycyanide (R=4-CONHC₆H₅phenyl)

4-(N-phenylamido)nitrosobenzene (2.0 g, 8.8 mmol) was suspended in dimethylformamide (20 ml) and N-bromosuccinimide (2.4 g, 13.5 mmol) was added. At 20-25°C a solution of cyanamide (0.56 g, 13.3 mmol) and sodium hydroxide (0.51 g, 12.8 mmol) in water (3 ml) was added drop by drop over a period of 10 minutes. After 1 hour, the suspension was poured on ice-water (100 ml). The resulting solid was filtered, washed with water and dried to give 2.1 g [4-(N-phenylamido)phenyl]-ONN-azoxycyanide as a yellow powder, m.pt. 220-225°C (Yield: 89.6% of theoretical).

### Example 4

### Preparation of 4-(N-acetylamino)phenyl-ONN-azoxycyanide (R=4-NHCOCH₃ phenyl)

4-(N-acetylamino)nitrosobenzene (15.0 g, 91.5 mmol) and N-bromosuccinimide (16.3, 91.6 mmol) were dissolved in dimethylformamide (150 ml). At ambient temperature, the solution was treated with monosodium cyanamide (9.14 g, 142.8 mmol). After 45 minutes, the brown solution was treated with N-bromosuccinimide (6.4 g, 36 mmol) and stirred for an additional 1½ hours. The mixture was poured on ice-water (500 ml) and left overnight. The yellow crystallised precipitate was collected and dried to give 16.8 g 4-(N-acetylamino)phenyl-ONN-azoxycyanide as a yellow powder, m.pt. 240-242°C (Yield: 90% of theoretical).

### Example 5

### Preparation of (4-N-acetylamino)phenyl-ONN-azoxycyanide (R=4-NHCOCH₃phenyl)

4-(N-acetylamino)nitrosobenzene (1.64 g, 10.0 mmol) was dissolved in dimethylformamide (20 ml) and treated with monosodium cyanamide (1.0 g, 15.6 mmol). N-chlorosuccinimide (1.33 g, 10.0 mmol) was added and the mixture stirred for 24 hours at ambient temperature. The mixture was poured on ice-water (100 ml) and the precipitate filtered off to give 1.2 g 4-(N-acetylamino)phenyl-ONN-azoxycyanide as a yellow powder, m.pt. 240-242°C (Yield: 58.8% of theoretical).

### Example 6

### Preparation of 1,3-benzodioxanyl-6-ONN-azoxycyanide (R=1,3-benzodioxan-6-yl)

6-Nitroso-1,3-benzodioxane (1.0 g, 6.1 mmol) and N-bromosuccinimide (1.1g, 6.2 mmol) were dissolved in dimethylformamide (20 ml). Monosodium cyanamide (0.61 g, 9.5 mmol) was added. The mixture was stirred for 1 hour at ambient temperature and poured on ice-water (80 ml), filtered, washed with water and dried to give 0.9 g 1,3-benzodioxanyl-6-ONN-azoxycyanide as a yellow powder, m.pt. 156-158°C. (Yield: 71.9% of theoretical).

### Example 7

### Preparation of 3,4-methylenedioxyphenyl-ONN-azoxycyanide (R=3,4-OCH₂O-phenyl)

3,4-Methylenedioxynitrosobenzene (1.05 g, 6.9 mmol) was dissolved in dichloromethane (20 ml). After the addition of cyanamide (0.42 g, 9.9 mmol) and N-bromosuccinimide (1.8 g, 9.9 mmol) the mixture was stirred at ambient temperature overnight. The resulting mixture was filtered and evaporated to dryness. Column chromatography on silica using 1:1 petroleum ether: ethyl acetate as eluant yielded 1.1 g 3,4-methylenedioxyphenyl-ONN-azoxycyanide as a yellow solid, m.pt. 145-147°C. (Yield: 82.7% of theoretical).

## Claims

1. A process for the preparation of a compound of the general formula 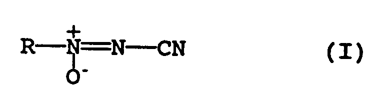 in which R represents an optionally substituted aryl or heterocyclyl group, which comprises treating a compound of the general formula
R-N=O (II)
in which R is as defined above, with cyanamide or a metal salt thereof and a compound of the general formula 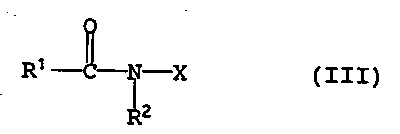 in which R¹ represents an optionally substituted alkyl or aryl group and R² represents a hydrogen atom or an optionally substituted alkyl or aryl group or R¹ and R² together represent a group 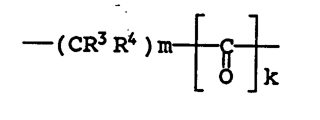 where m is 2, 3, 4 or 5, k is 0 or 1 and each of R³ and R⁴ independently represents a hydrogen atom or an alkyl group, and X represents a chlorine, bromine or iodine atom or a cyano or -SO₂R⁵ group where R⁵ represents a hydrogen atom or an optionally substituted alkyl or aryl group.

2. A process according to claim 1 in which R represents a substituted phenyl group in which at least one substituent thereof is a group of general formula -NR⁶COR⁷ where R⁶ represents a hydrogen atom or an alkyl group and R⁷ represents a hydrogen atom or an optionally substituted alkyl, alkoxy, alkenyl, alkenyloxy, phenyl or phenoxy group.

3. a process according to claim 1 in which R represents a substituted phenyl group in which at least one substituent thereof is an amido group of general formula -CONR⁸R⁹ where each of R⁸ and R⁹ independently represents a hydrogen atom or an optionally substituted alkyl or phenyl group.

4. A process according to claim 1 in which R represents a group of general formula 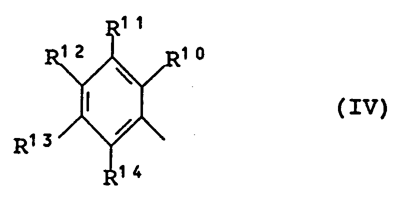 in which R¹⁰ and R¹¹ together, or R¹¹ and R¹² together, represent an optionally substituted hydrocarbyloxy chain and the ring is optionally substituted at any or each of the remaining sites R¹³, R¹⁴ and R¹⁰ or R¹².

5. A process according to claim 1 in which R represents a group of general formula 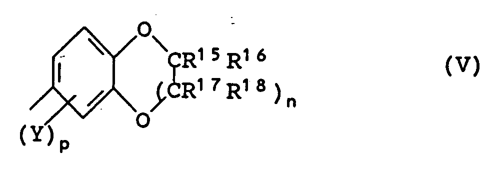 in which n is 0 or 1; each of R¹⁵ and R¹⁶, and R¹⁷ and R¹⁸ if present, independently represents a hydrogen or halogen atom or an optionally substituted alkyl, cycloalkyl or aryl group, or R¹⁵ and R¹⁶ together or R¹⁷ and R¹⁸ together represent an optionally substituted alkylene chain; Y represents an alkyl group or a halogen atom; and p represents 0, 1, 2 or 3.

6. A process according to claim 1 in which R represents a substituted phenyl group in which at least one substituent thereof is a group of general formula S(O)_{q}Z where q represents 0, 1 or 2 and Z represents a hydrogen atom or an optionally substituted alkyl, cycloalkyl, aryl, aralkyl or amino group.

7. A process according to claim 1 in which R represents a group of general formula 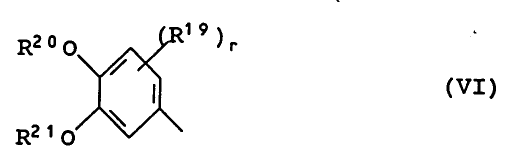 in which r is 0, 1, 2 or 3; each R¹⁹ independently represents a halogen atom, nitro, cyano, alkyl, haloalkyl, alkoxy or haloalkoxy group; and each of R²⁰ and R²¹ independently represents an optionally substituted alkyl, alkenyl, alkynyl, cycloalkyl or aralkyl group.

8. A process according to claim 1 in which R represents a group of general formula 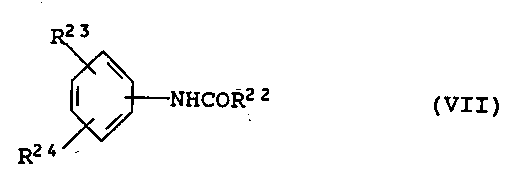 in which R²² represents an optionally substituted alkyl, alkenyl, alkynyl, cycloalkyl, cycloalkenyl, aryl or heterocyclyl group; R²³ represents a halogen atom, nitro, cyano or optionally substituted alkyl, alkoxy, aryl or aryloxy group; and R²⁴ represents a hydrogen or halogen atom, nitro, cyano or optionally substituted alkyl or alkoxy group.

9. A process according to claim 1 in which R represents a group of general formula 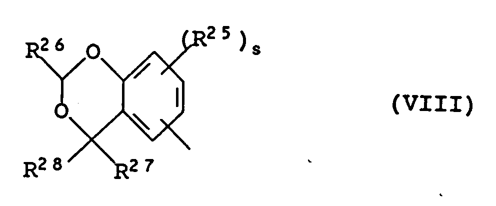 in which s is 0, 1 or 2; each R²⁵, if present, independently represents a halogen atom or an optionally substituted alkyl or alkoxy group; R²⁶ represents a hydrogen atom or a haloalkyl group; and R²⁷ and R²⁸ independently represent a hydrogen atom or an alkyl or haloalkyl group.

10. A process according to any one of the preceding claims in which monosodium cyanamide is used.

11. A process according to any one of the preceding claims in which R¹ and R² together represent a group

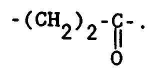

12. A process according to any one of the preceding claims in which X is a chlorine or bromine atom.

13. A process according to any one of the preceding claims in which the compound of formula III is N-bromosuccinimide.

14. A process according to any one of the preceding claims in which the process is carried out at a temperature in the range from -5°C to 35°C.

## Patentansprüche

1. Verfahren zur Herstellung einer Verbindung der allgemeinen Formel 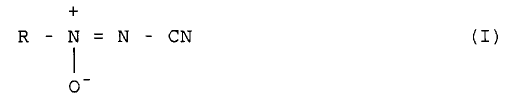 worin R eine gegebenenfalls substituierte Aryl- oder Heterocycylgruppe wiedergibt, umfassend Umsetzen einer Verbindung der allgemeinen Formel
R - N = O (II)
worin R wie vorstehend definiert ist, mit Cyanamid oder einem Metallsalz davon und einer Verbindung der allgemeinen Formel 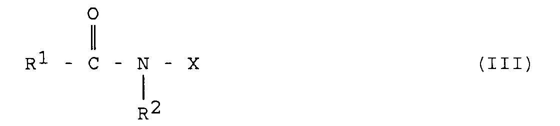 worin R¹ eine gegebenenfalls substituierte Alkyl- oder Arylgruppe wiedergibt, und R² ein Wasserstoffatom oder eine gegebenenfalls substituierte Alkyl- oder Arylgruppe wiedergibt oder R¹ und R² zusammen eine Gruppe 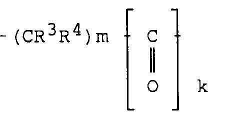 darstellen, worin m 2, 3, 4 oder 5 ist, k 0 oder 1 ist und jeder der Reste R³ und R⁴ unabhängig ein Wasserstoffatom oder eine Alkylgruppe wiedergibt und X ein Chlor-, Brom- oder Jodatom oder eine Cyano- oder eine Gruppe -SO₂R⁵ wiedergibt, worin R⁵ ein Wasserstoffatom oder eine gegebenenfalls substituierte Alkyl- oder Arylgruppe wiedergibt.

2. Verfahren nach Anspruch 1, wobei R eine substituierte Phenylgruppe wiedergibt, worin mindestens ein Substituent davon eine Gruppe der allgemeinen Formel -NR⁶COR⁷ wiedergibt, in der R⁶ ein Wasserstoffatom oder eine Alkylgruppe wiedergibt und R⁷ ein Wasserstoffatom oder eine gegebenenfalls substituierte Alkyl-, Alkoxy-, Alkenyl-, Alkenyloxy-, Phenyl- oder Phenoxygruppe wiedergibt.

3. Verfahren nach Anspruch 1, wobei R eine substituierte Phenylgruppe wiedergibt, worin mindestens ein Substituent davon eine Amidogruppe der allgemeinen Formel -CONR⁸R⁹ wiedergibt, in der jeder der Reste R⁸ und R⁹ unabhängig ein Wasserstoffatom oder eine gegebenenfalls substituierte Alkyl- oder Phenylgruppe wiedergibt.

4. Verfahren nach Anspruch 1, wobei R eine Gruppe der allgemeinen Formel 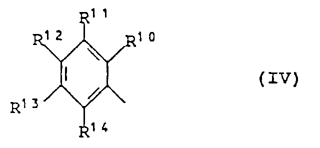 wiedergibt, worin R¹⁰ und R¹¹ zusammengenommen oder R¹¹ und R¹² zusammengenommen eine gegebenenfalls substituierte Kohlenwasserstoffoxykette wiedergeben und der Ring gegebenenfalls an einer oder jeder der verbleibenden Stellen R¹³, R¹⁴ und R¹⁰ oder R¹² substituiert ist.

5. Verfahren nach Anspruch 1, wobei R eine Gruppe der allgemeinen Formel 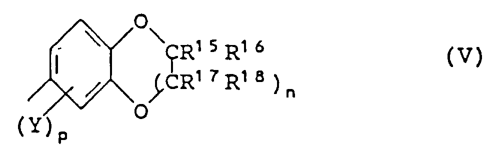 wiedergibt, worin n 0 oder 1 ist; jeder der Reste R¹⁵ und R¹⁶, und R¹⁷ und R¹⁸, falls vorliegend, unabhängig ein Wasserstoff- oder ein Halogenatom oder eine gegebenenfalls substituierte Alkyl-, Cycloalkyl- oder Arylgruppe wiedergibt, oder R¹⁵ und R¹⁶ zusammengenommen oder R¹⁷ und R¹⁸ zusammengenommen eine gegebenenfalls substituierte Alkylenkette wiedergeben; Y eine Alkylgruppe oder ein Halogenatom wiedergibt; und p 0, 1, 2 oder 3 ist.

6. Verfahren nach Anspruch 1, wobei R eine substituierte Phenylgruppe wiedergibt, worin mindestens ein Substituent davon eine Gruppe der allgemeinen Formel -S(O)_{q}Z darstellt, in der q 0, 1 oder 2 wiedergibt und Z ein Wasserstoffatom oder eine gegebenenfalls substituierte Alkyl-, Cycloalkyl-, Aryl, Aralkyl- oder Aminogruppe wiedergibt.

7. Verfahren nach Anspruch 1, wobei R eine Gruppe der allgemeinen Formel 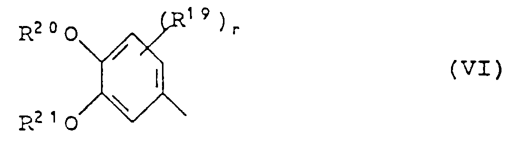 wiedergibt, worin r 0, 1, 2 oder 3 ist; jeder der Reste R¹⁹ unabhängig ein Halogenatom, eine Nitro-, Cyano-, Alkyl-, Halogenalkyl-, Alkoxy- oder Halogenalkoxygruppe wiedergibt; und jeder der Reste R²⁰ und R²¹ unabhängig eine gegebenenfalls substituierte Alkyl-, Alkenyl-, Alkinyl, Cycloalkyl- oder Aralkylgruppe wiedergibt.

8. Verfahren nach Anspruch 1, wobei R eine Gruppe der allgemeinen Formel 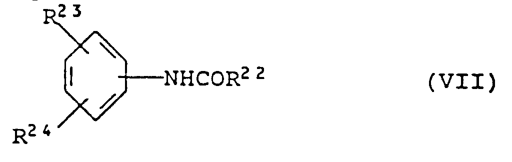 wiedergibt, worin R²² eine gegebenenfalls substituierte Alkyl-, Alkenyl-, Alkinyl-, Cycloalkyl-, Cycloalkenyl-, Aryl- oder Heterocyclylgruppe wiedergibt; R²³ ein Halogenatom, eine Nitro-, Cyano- oder gegebenenfalls substituierte Alkyl-, Alkoxy-, Aryl- oder Aryloxygruppe wiedergibt; und R²⁴ ein Wasserstoff- oder Halogenatom, eine Nitro-, Cyano- oder gegebenenfalls substituierte Alkyl- oder Alkoxygruppe wiedergibt.

9. Verfahren nach Anspruch 1, wobei R eine Gruppe der allgemeinen Formel 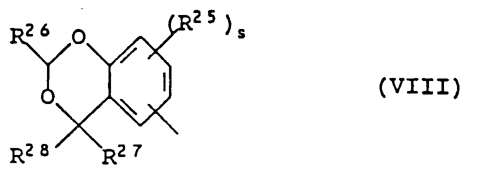 wiedergibt, worin s 0, 1 oder 2 ist; jeder der Reste R²⁵, falls vorliegend, unabhängig ein Halogenatom oder eine gegebenenfalls substituierte Alkyl- oder Alkoxygruppe wiedergibt; R²⁶ ein Wasserstoffatom oder eine Halogenalkylgruppe wiedergibt; und R²⁷ und R²⁸ unabhängig ein Wasserstoffatom oder eine Alkyl- oder Halogenalkylgruppe wiedergeben.

10. Verfahren nach einem der vorangehenden Ansprüche, wobei Mononatriumcyanamid verwendet wird.

11. Verfahren nach einem der vorangehenden Ansprüche, wobei R¹ und R² zusammen eine Gruppe 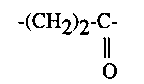 wiedergeben.

12. Verfahren nach einem der vorangehenden Ansprüche, wobei X ein Chlor- oder Bromatom darstellt.

13. Verfahren nach einem der vorangehenden Ansprüche, wobei die Verbindung der Formel III N-Bromsuccinimid darstellt.

14. Verfahren nach einem der vorangehenden Ansprüche, wobei das Verfahren bei einer Temperatur im Bereich -5°C bis 35°C ausgeführt wird.

## Revendications

1. Procédé pour la préparation d'un composé de formule générale 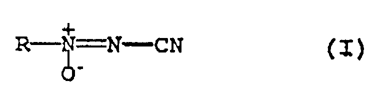 dans laquelle R représente un groupe aryle ou un groupe hétérocyclique éventuellement substitué, comprenant le traitement d'un composé de formule générale
R-N=O (II)
dans laquelle R est tel que défini ci-dessus, avec un cyanamide ou un sel métallique de celui-ci et un composé de formule générale 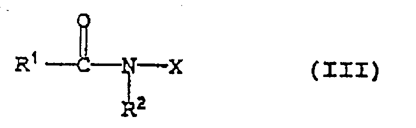 dans laquelle R¹ représente un groupe alkyle ou aryle éventuellement substitué et R² représente un atome d'hydrogène ou un groupe alkyle ou aryle éventuellement substitué ou bien R¹ et R² représentent ensemble un groupe 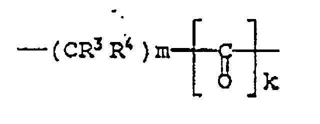 dans lequel m est 2, 3, 4 ou 5, k est 0 ou 1 et R³ et R⁴ représentent indépendamment un atome d'hydrogène ou un groupe alkyle, et X représente un atome de chlore, de brome ou d'iode ou un groupe cyano ou un groupe -SO₂R⁵ dans lequel R⁵ représente un atome d'hydrogène ou un groupe alkyle ou aryle éventuellement substitué.

2. Procédé selon la revendication 1, dans lequel R représente un groupe phényle substitué dans lequel au moins un des substituants du groupe phényle est un groupe de formule générale -NR⁶COR⁷ dans lequel R⁶ représente un atome d'hydrogène ou un groupe alkyle et R⁷ représente un atome d'hydrogène ou un groupe alkyle, alcoxy, alcényle, alcényloxy, phényle ou phénoxy éventuellement substitué.

3. Procédé selon la revendication 1, dans lequel R représente un groupe phényle substitué dans lequel au moins un des substituants du phényle est un groupe amido de formule générale -CONR⁸R⁹ dans lequel R⁸ et R⁹ représentent indépendamment un atome d'hydrogène ou un groupe alkyle ou phényle éventuellement substitué.

4. Procédé selon la revendication 1 dans lequel R représente un groupe de formule générale 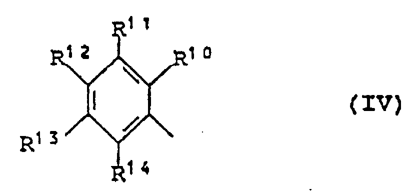 dans laquelle R¹⁰ et R¹¹ ou bien R¹¹ et R¹² représentent ensemble une chaîne hydrocarbyloxy éventuellement substituée et le noyau est éventuellement substitué à l'une quelconque ou en chacune des positions restantes R¹³, R¹⁴ et R¹⁰ ou R¹².

5. Procédé selon la revendication 1 dans lequel R représente un groupe de formule générale 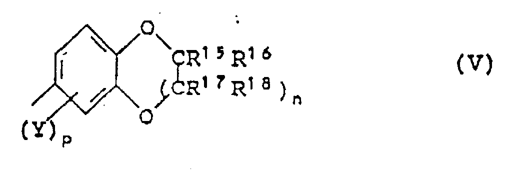 dans laquelle n est 0 ou 1; R¹⁵ et R¹⁶, et R¹⁷ et R¹⁸ dans le cas où ils sont présents, représentent indépendamment un atome d'hydrogène ou un atome d'halogène ou un groupe alkyle, cycloalkyle ou aryle éventuellement substitué, ou bien R¹⁵ et R¹⁶ ou R¹⁷ et R¹⁸ représentent ensemble une chaîne alkylène éventuellement substituée; Y représente un groupe alkyle ou un atome d'halogène; et p représente 0, 1, 2 ou 3.

6. Procédé selon la revendication 1, dans lequel R représente un groupe phényle substitué dans lequel au moins un des substituants du groupe phényle est un groupe de formule générale -S(O)_{q}Z dans lequel q représente 0, 1 ou 2 et Z représente un atome d'hydrogène ou un groupe alkyle, cycloalkyle, aryle, aralkyle ou amino éventuellement substitué.

7. Procédé selon la revendication 1, dans lequel R représente un groupe de formule générale 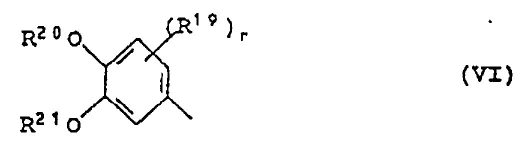 dans lequel r est 0, 1, 2 ou 3; R¹⁹ représentent indépendamment un atome d'halogène, un groupe nitro, cyano, alkyle, halogénoalkyle, alkoxy ou halogénoalkoxy; et R²⁰ et R²¹ représentent indépendamment un groupe alkyle, alcényle, alcynyle, cycloalkyle ou aralkyle éventuellement substitué.

8. Procédé selon la revendication 1, dans lequel R représente un groupe de formule générale 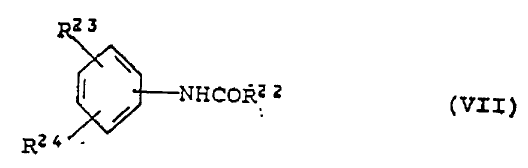 dans laquelle R²² représente un groupe alkyle, alcényle, alcynyle, cycloalkyle, cycloalcényle, aryle ou hétérocyclyle éventuellement substitué; R²³ représente un atome d'halogène, un groupe nitro, cyano ou un groupe alkyle, alkoxy, aryle ou aryloxy éventuellement substitué; et R²⁴ représente un atome d'hydrogène ou un atome d'halogène, un groupe nitro, cyano ou un groupe alkyle ou alkoxy éventuellement substitué.

9. Procédé selon la revendication 1, dans lequel R représente un groupe de formule générale 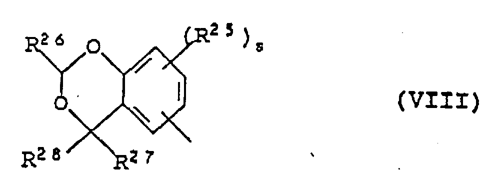 dans laquelle s est 0, 1 ou 2; R²⁵ lorsqu'il est présent, représente indépendamment un atome d'halogène ou un groupe alkyle ou alkoxy éventuellement substitué; R²⁶ représente un atome d'hydrogène ou un groupe halogénoalkyle et R²⁷ et R²⁸ représentent indépendamment un atome d'hydrogène ou un groupe alkyle ou halogénoalkyle.

10. Procédé selon l'une quelconque des revendications précédentes, dans lequel on utilise du cyanamide monosodique.

11. Procédé selon l'une quelconque des revendications précédentes, dans lequel R¹ et R² représentent ensemble un groupe :

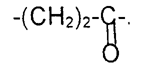

12. Procédé selon l'une quelconque des revendications précédentes, dans lequel X est un atome de chlore ou de brome.

13. Procédé selon l'une quelconque des revendications précédentes, dans lequel le composé de formule III est le N-bromosuccinimide.

14. Procédé selon l'une quelconque des revendications précédentes, dans lequel le procédé est mis en oeuvre à une température comprise entre -5° C et 35° C.
